# EUROPEAN PATENT APPLICATION

(11) **EP 1 777 298 A1**
(43) Date of publication of application: **25.04.2007**
(21) Application number: 06021285.9
(22) Date of filing: 11.10.2006
(51) Int. Cl.: C12Q 1/68

(54) **Multiplexed solid-phase nucleic acid amplification assay**

(30) Priority: 13.10.2005 US 726977 P
(71) Applicant: Roche Diagnostics GmbH, 68305 Mannheim (DE); F. HOFFMANN-LA ROCHE AG, 4070 Basel (CH)
(72) Inventor: Pollart, Daniel, Alameda, California 94502 (US); Will, Stephen Gordon, Oakland, California 94611 (US)

(57) **Abstract**

The present invention provides methods for detecting the amplification of a target nucleic acid using an oligonucleotide probe immobilized onto a solid substrate via its 3'-end. The invention further contemplates an apparatus, reaction mixtures and kits for carrying out the present methods.

## Description

### FIELD OF THE INVENTION

The present invention is within the field of nucleic acid detection.

### BACKGROUND OF THE INVENTION

The use of solution phase dual-labeled oligonucleotide probes employed in conjunction with an enzyme having 5'-nuclease activity to detect the amplification of a nucleic acid sequence is known in the art *(see,* for example, U.S. Patent Nos. 5,210,015; 5,487,972; 5,804,375; and Holland, et al., Proc. Natl. Acad. Sci. USA (1988) 88:7276-80). Until the present invention, immobilized dual-labeled oligonucleotide probes bearing label moieties releasable by 5'-nuclease enzyme activity have not been used during nucleic acid amplification reactions or for the real-time monitoring of nucleic acid amplification reactions.

There remains a need for more efficient and more convenient methods to simultaneously detect the amplification of multiple nucleic acid sequences. The present invention fulfills this and other needs.

### BRIEF SUMMARY OF THE INVENTION

In one aspect, the present invention provides a method for detecting a target nucleic acid, the method comprising the steps of:
a) providing an oligonucleotide probe immobilized onto a solid substrate via its 3'-end, the probe comprising a label moiety;
b) amplifying a nucleic acid sample suspected of comprising the target nucleic acid in a reaction mixture containing an enzyme having 5'-3'-nuclease activity in the presence of the probe, wherein the probe specifically hybridizes to the target nucleic acid such that the enzyme having 5'-3'-nuclease activity releases the label moiety from the oligonucleotide probe and the presence or absence of the target nucleic acid in the nucleic acid sample is detected.

The invention further provides a method of simultaneously detecting multiple target nucleic acid sequences, the method comprising the steps of:
a) providing a plurality of oligonucleotide probes, each probe immobilized onto a solid substrate via its 3'-end, each probe comprising the same label moiety;
b) amplifying multiple nucleic acid samples suspected of comprising a target nucleic acid in a reaction mixture containing an enzyme having 5'-3'-nuclease activity in the presence of the probe, wherein each probe specifically hybridizes to one of the multiple target nucleic acids such that the enzyme having 5'-3'-nuclease activity releases the label moiety from the oligonucleotide probes and the presence or absence of the target nucleic acids in the nucleic acid samples is detected. In this method detection of fluorescence indicative of a target nucleic acid is spatially distinguished. Using the present methods, at least 8, 10, 12, 14, 15, 16, 18, 20, 50, 75, 100 or more target nucleic acid sequences can be simultaneously detected.

In one embodiment, the label moiety is a fluorophore moiety attached to the 5'-end of the probe, whereby release of the fluorophore moiety results in a decrease in the fluorescence on the immobilized probe, thereby detecting the presence of the target nucleic acid in the nucleic acid sample.

In one embodiment, the label moiety is a quencher moiety attached to the 5'-end of the probe and the immobilized probe further comprises a fluorophore moiety attached to the probe 3' from the quencher moiety, wherein the fluorophore moiety does not fluoresce when the quencher moiety is attached to the probe, and the fluorescence of the fluorophore moiety on the immobilized probe increases with increasing amplification of the target nucleic acid as the quencher moiety is released into solution.

In one embodiment, the label moiety is a quencher moiety attached to the 5'-end of the probe and the fluorescence of a fluorophore moiety immobilized adjacent to the probe increases with increasing amplification of the target nucleic acid. For example, the fluorophore moiety can be immobilized on the solid substrate or on a second nucleic acid *(i.e.,* a second probe) immobilized to the solid substrate sufficiently close to the probe bearing the quencher moiety to mask the fluorescence of the fluorophore.

In one embodiment, amplifying is by polymerase chain reaction. In one embodiment, the enzyme having 5'-3'-nuclease activity is a DNA polymerase having 5'-3'-nuclease activity.

The solid substrate can be any material that can withstand intermittent and repeated nucleic acid denaturation temperatures *(i.e.,* 80°C-105°C, usually about 95°C) and remain bound to the immobilized probe. Preferred substrates allow for the simultaneous detection of multiple nucleic acid sequences. In the different embodiments of the present invention, the solid substrate can be, for example, a multiwell plate, a microscope slide, a polymerase chain reaction (PCR) tube, or a flat-surfaced disk. The solid substrate can be any polygonal shape, for example, a circle, an oval, a square, a rectangle, a triangle, a hexagon, and the like.

In some embodiments the amplification detection is quantitative. In some embodiments, the amplification detection is concurrent with amplification (i.e., "real-time"). In some embodiments the amplification detection is quantitative, and the amplification detection is real-time. In some embodiments, the amplification detection is qualitative (e.g., end-point detection of the presence or absence of a target nucleic acid). In some embodiments, the amplification detection is subsequent to amplification. In some embodiments, the amplification detection is qualitative, and the amplification detection is subsequent to amplification.

In some embodiments, the fluorophore moiety is selected from the group consisting of fluorescein-family dyes, polyhalofluorescein-family dyes, hexachlorofluorescein-family dyes, coumarin-family dyes, rhodamine-family dyes, cyanine-family dyes, oxazine-family dyes, thiazine-family dyes, squaraine-family dyes, chelated lanthanide-family dyes, azo-family dyes, triphenylmethane-family dyes, and BODIPY^{®}-family dyes.

In some embodiments, the quencher moiety is selected from the group consisting of Iluorescein-family dyes, polyhalofluorescein-family dyes, hexachlorofluorescein-family dyes, coumarin-family dyes, rhodamine-family dyes, cyanine-family dyes, oxazine-family dyes, thiazine-family dyes, squaraine-family dyes, chelated lanthanide-family dyes, BODIPY®-family dyes, azo-family dyes, triphenylmethane-family dyes, low-fluorescent quencher moieties (i.e., "dim donors") and non-fluorescent quencher moieties (e.g., so-called "dark quenchers" including Black Hole Quenchers™ (BHQ)).

In one embodiment, the fluorophore moiety is a fluorescein-family dye and the quencher moiety is a cyanine-family dye. In one embodiment, the fluorophore moiety is a fluorescein-family dye and the quencher moiety is a hexachlorofluorescein-family dye. In one embodiment, the fluorophore moiety is a hexachlorofluorescein-family dye and the quencher moiety is a cyanine-family dye. In one embodiment, the quencher is a dark quencher, for example, a BHQ-1, a BHQ-2 or a BHQ-3 (Biosearch Technologies, Novato, CA). In one embodiment, the fluorophore moiety is a fluorescein-family dye or a cyanine-family dye and the quencher moiety is a dark quencher.

In a further aspect, the invention provides a reaction mixture comprising:
i) an oligonucleotide probe immobilized to a solid substrate via its 3'-end, the probe comprising a label moiety that is released by an enzyme having 5'-3'-nuclease activity; and
ii) reagents for amplification of a target nucleic acid, wherein the probe hybridizes to a sequence within the target nucleic acid,

In a further aspect, the invention provides a kit comprising:
i) an oligonucleotide probe immobilized to a solid substrate via its 3'-end, the probe comprising a label moiety that is released by an enzyme having 5'-3'-nuclease activity; and
ii) reagents for amplification of a target nucleic acid, wherein the probe hybridizes to the target nucleic acid.

The invention further provides for a solid substrate comprising at least one oligonucleotide probe immobilized to the solid substrate via its 3'-end, the at least one probe comprising a label moiety that is released by an enzyme having 5'-3'-nuclease activity.

The invention also contemplates an apparatus for carrying out amplification of a target nucleic acid, the apparatus comprising at least one oligonucleotide probe immobilized to a solid substrate via its 3'-end, the at least one probe comprising a label moiety that is released by an enzyme having 5'-3'-nuclease activity.

The invention further contemplates an apparatus for carrying out amplification of a target nucleic acid, the apparatus comprising a space to accommodate a solid substrate comprising at least one oligonucleotide probe immobilized to the solid substrate via its 3'-end, the at least one probe comprising a label moiety that is released by an enzyme having 5'-3'-nuclease activity.

The embodiments for the reaction mixtures, kits and apparatus are the same as for the methods.

### BRIEF DESCRIPTION OF THE DRAWINGS

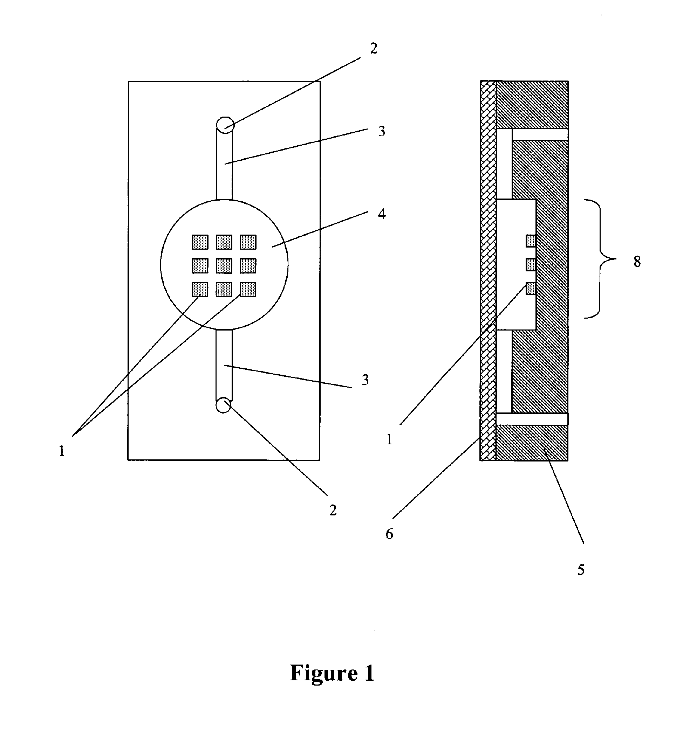

Figure 1 illustrates a solid substrate flat-surfaced disk array on body. (1) array member; (2) fluidic port; (3) channel; (4) chamber; (5) body; (6) sealing wall; (7) array substrate; and (8) optical window.

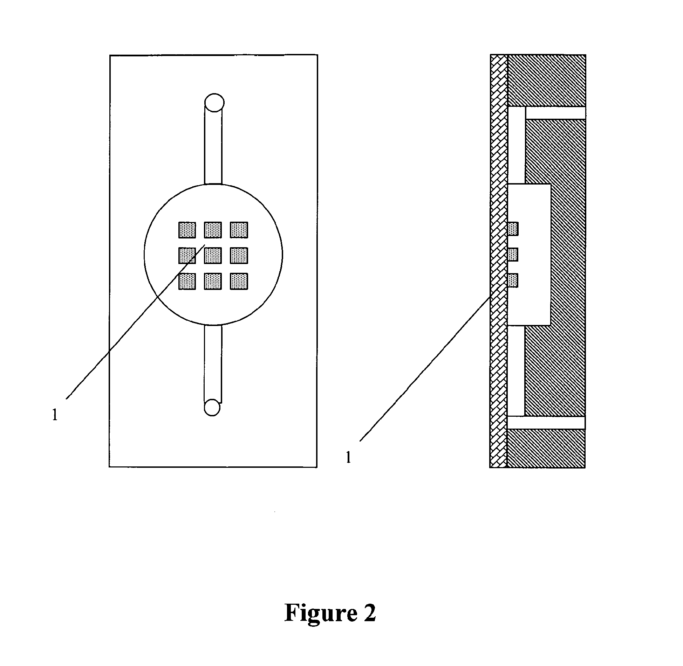

Figure 2 illustrates a solid substrate flat-surfaced disk array on sealing wall.

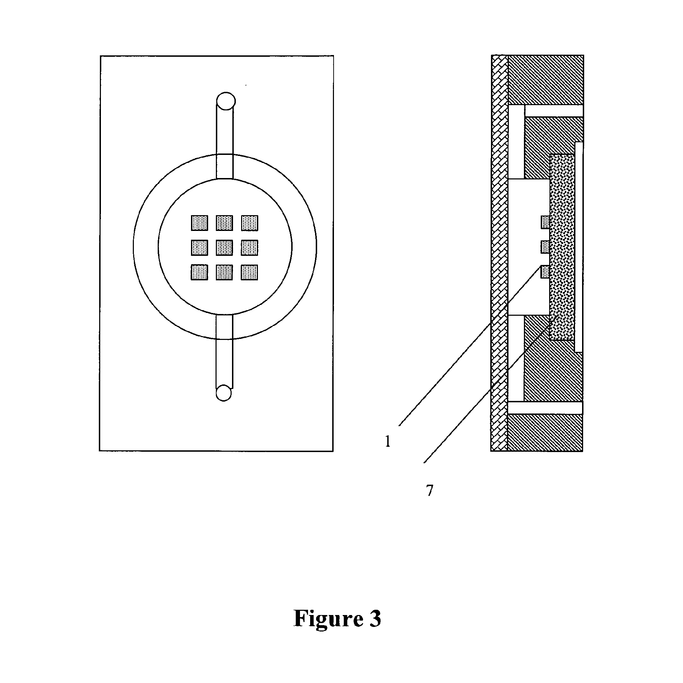

Figure 3 illustrates a solid substrate flat-surfaced disk array, assembled.

### DETAILED DESCRIPTION

### 1. Introduction

As shown herein, little or no modification of polymerase chain reaction (PCR) thermocycling parameters is required to achieve an amplification growth curve using immobilized dual-labeled probes. Further, measurements of cycle threshold (Ct) values acquired from immobilized dual-labeled probes are similar to those acquired with solution phase dual-labeled probes. Capture of a target nucleic acid by an immobilized probe occurs at a rate that is competitive with a solution phase amplification reaction.

As shown below, immobilization of the oligonucleotide probes allows for the simultaneous real-time analysis of numerous nucleic acid amplification reactions. Current multiplexed analyses require the use of multiple dye and quencher combinations, and are generally limited by the excitation and detection systems available on a single instrument to about six simultaneous reactions per sample. With the present invention, multiplexing can be achieved with the use of a single dye and quencher combination through the spatial distribution of dual-labeled oligonucleotide probes in a two-dimensional array. The number of analyses per sample is therefore limited by the ability to multiplex amplification reactions and design sufficiently selective probes. Using the present invention multiple simultaneous reactions can be monitored at one time in a single reaction.

### 2. Abbreviations

The abbreviations used throughout the specification to refer to nucleic acids comprising specific nucleotide sequences are the conventional one-letter abbreviations. Thus, when included in DNA, the naturally occurring deoxyribonucleosides encoding nucleotides are abbreviated as follows: deoxyadenosine (dA), deoxyguanosine (dG), deoxycytidine (dC) and deoxythymidine (dT). Ribonucleosides are abbreviated as follows: adenosine (A), guanosine (G), cytidine (C), and uridine (U). Nucleotide bases are abbreviated as follows: adenine (A), guanine (G), cytosine (C), thymine (T) and uracil (U). Also, unless otherwise specified, nucleic acid sequences that are represented as a series of one-letter abbreviations are presented in the 5' -> 3' direction.

### 3. Definitions

An "amplification reaction" refers to any reaction (e.g., chemical, enzymatic, or other type of reaction) that results in increased copies of a template nucleic acid sequence or increased signal indicating the presence of the template. Amplification reactions include, e.g., the polymerase chain reaction (PCR) and ligase chain reaction (LCR) *(see* U.S. Patents 4,683,195 and 4,683,202; PCR Protocols: A Guide to Methods and Applications (Innis et al., eds, 1990)), strand displacement amplification (SDA) (Walker, et al. Nucleic Acids Res. 20(7):1691-6 (1992); Walker PCR Methods Appl 3(1):1-6 (1993)), transcription-mediated amplification (Phyffer, et al., J. Clin. Microbiol. 34:834-841 (1996); Vuorinen, et al. , J. Clin. Microbiol. 33:1856-1859 (1995)), nucleic acid sequence-based amplification (NASBA) (Compton, Nature 350(6313):91-2 (1991), rolling circle amplification (RCA) (Lisby, Mol. Biotechnol. 12(1):75-99 (1999)); Hatch et al., Genet. Anal. 15(2):35-40 (1999)) and Q-Beta Replicase (Lizardi et al., BiolTechnology 6:1197 (1988)).

As used herein, "reagents for amplification" or "amplification reagents" interchangeably refer to the reagents commonly included by those in the art in a reaction vessel for an amplification reaction. For example, a typical polymerase chain reaction can include, without limitation, as reagents upstream and downstream primers, at least one template, deoxyribonucleoside triphosphates (including dATP, dCTP, dGTP, TTP, dUTP), a polymerase enzyme, buffers, metal cations and salts. A nucleic acid sequence-based amplification (NASBA) reaction can include primers, reverse transcriptase, RNase H, and a DNA polymerase. A transcription-mediated amplification (TMA) reaction can include primers, reverse transcriptase, and an RNA polymerase. An strand displacement amplification (SDA) reaction can include a modified nucleotide and a restriction endonuclease. Certain amplification reactions can also include deoxyUridine N-Glycosylase (UNG) as an ancillary amplification reagent (*e.g.*, Amperase^{®}, Roche Molecular Sciences, Alameda, CA) (*see,* Kleiboeker, *Virol J* (2005) 11:29).

As used herein, a "sample" refers to any substance containing or presumed to contain a target nucleic acid. The sample can be of natural or synthetic origin and can be obtained by any means known to those of skill in the art. The sample can be a sample of tissue or fluid isolated from an individual or individuals, including, but not limited to, for example, skin, plasma, serum, whole blood, spinal fluid, semen, seminal fluid, lymph fluid, synovial fluid, urine, tears, blood cells, organs, tumors, bronchio-alveolar lavage, and also to samples of in vitro cell culture constituents (including but not limited to conditioned medium resulting from the growth of cells in cell culture medium, recombinant cells and cell components). A nucleic acid can be obtained from a biological sample by any procedure known in the art.

As used herein, the terms "nucleic acid," "polynucleotide" and "oligonucleotide" refer to primers, probes, oligomer fragments to be detected, oligomer controls and unlabeled blocking oligomers and is generic to linear polymers of polydeoxyribonucleotides (containing 2-deoxy-D-ribose), polyribonucleotides (containing D-ribose), and any other N-glycoside analogs of a purine or pyrimidine base, or modified purine or pyrimidine bases.

A nucleic acid, polynucleotide or oligonucleotide can comprise phosphodiester linkages or modified linkages including, but not limited to phosphotriester, phosphoramidate, siloxane, carbonate, carboxymethylester, acetamidate, carbamate, thioether, bridged phosphoramidate, bridged methylene phosphonate, phosphorothioate, methylphosphonate, phosphorodithioate, bridged phosphorothioate or sulfone linkages, and combinations of such linkages.

A nucleic acid, polynucleotide or oligonucleotide can comprise the five biologically occurring bases (adenine, guanine, thymine, cytosine and uracil) and/or bases other than the five biologically occurring bases. These bases may serve a number of purposes, e.g., to stabilize or destabilize hybridization; to promote or inhibit probe degradation; or as attachment points for detectable moieties or quencher moieties. For example, a polynucleotide of the invention can contain one or more modified, non-standard, or derivatized base moieties, including, but not limited to, N⁶-methyl-adenine, N⁶-tert-butyl-benzyl-adenine, imidazole, substituted imidazoles, 5-fluorouracil, 5-bromouracil, 5-chlorouracil, 5-iodouracil, hypoxanthine, xanthine, 4-acetylcytosine, 5-(carboxyhydroxymethyl)uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-methyladenine, 7-methylguanine, 5-methylaminomethyluracil, 5-methoxyaminomethyl-2-thiouracil, beta-D mannosylqueosine, 5'-methoxycarboxymethyluracil, 5-methoxyuracil, 2-methylthio-N6- isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil (i.e., thymine), uracil-5-oxyacetic acidmethylester, 3-(3-amino-3-N-2-carboxypropyl) uracil, (acp3)w, 2,6-diaminopurine, and 5-propynyl pyrimidine. Other examples of modified, non-standard, or derivatized base moieties may be found in U.S. Patent Nos. 6,001,611, 5,955,589, 5,844,106, 5,789,562, 5,750,343, 5,728,525, and 5,679,785.

Furthermore, a nucleic acid, polynucleotide or oligonucleotide can comprise one or more modified sugar moieties including, but not limited to, arabinose, 2-fluoroarabinose, xylulose, and a hexose.

It is not intended that the present invention be limited by the source of a nucleic acid, polynucleotide or oligonucleotide. A nucleic acid, polynucleotide or oligonucleotide can be from a human or non-human mammal, or any other organism, or derived from any recombinant source, synthesized in vitro or by chemical synthesis. A nucleic acid, nucleotide, polynucleotide or oligonucleotide may be DNA, RNA, cDNA, DNA-RNA, locked nucleic acid (LNA), peptide nucleic acid (PNA), a hybrid or any mixture of the same, and may exist in a double-stranded, single-stranded or partially double-stranded form. A nucleic acid may also be a derivative nucleic acid as described in U.S. Patent No. 5,696,248. The nucleic acids of the invention include both nucleic acids and fragments thereof, in purified or unpurified forms, including genes, chromosomes, plasmids, the genomes of biological material such as microorganisms, e.g., bacteria, yeasts, viruses, viroids, molds, fungi, plants, animals, humans, and the like.

There is no intended distinction in length between the terms nucleic acid, polynucleotide and oligonucleotide, and these terms will be used interchangeably. These terms include double- and single-stranded DNA, as well as double- and single-stranded RNA. Oligonucleotides of the invention may be used as primers and/or probes.

The term "residue" as used herein refers to a nucleotide or base within a nucleic acid as defined above. A residue can be any nucleotide known to one of skill in the art without limitation, including all of the biologically occurring nucleotides and non-biologically occurring nucleotides described above.

The term "primer" refers to an oligonucleotide which is capable of acting as a point of initiation of polynucleotide synthesis along a template nucleic acid strand when placed under conditions that permit synthesis of a primer extension product that is complementary to the template strand. The primer can be obtained from a recombinant source, as in a purified restriction fragment, or produced synthetically. Primer extension conditions typically include the presence of four different deoxyribonucleoside triphosphates and an agent with polymerization activity such as DNA polymerase or reverse transcriptase, in a suitable buffer (a "buffer" can include substituents which are cofactors, or which affect pH, ionic strength, etc.), and at a suitable temperature. The primer is preferably single-stranded for maximum efficiency in amplification. Primers of the invention may be, e.g., between 5 to 500 nucleotides, and in some embodiments will have at least 10, 20, 30, 25, 30, 40, 50, 75, or 100 nucleotides and/or have fewer than 500, 300, 200, 100, 90, 80, 70, 60, 50, 40, 30, 25, or 20 nucleotides.

The term "hybridize" refers to binding of a single-stranded nucleic acid or a locally single-stranded region of a double-stranded nucleic acid to another single-stranded nucleic acid or a locally single-stranded region of a double-stranded nucleic acid having a complementary sequence. As one of skill in the art is aware, it is not necessary for two nucleic acid strands to be entirely complementary to hybridize to each other.
Depending on the hybridization conditions, a nucleic acid can hybridize to its complement even if there are few, some, or many mismatches, deletions, or additions in one or both strands. In certain embodiments, the primers and probes of the invention can hybridize to an at least partially complementary nucleic acid selectively, as defined below. In certain embodiments, the primers and probes of the invention can hybridize to an at least partially complementary sequence under stringent conditions, as defined below.

The terms "stringent" or "stringent conditions", as used herein, denote hybridization conditions of low ionic strength and high temperature, as is well known in the art; see for example Maniatis et al., 1989, Molecular Cloning: A Laboratory Manual, 2d Edition; Current Protocols in Molecular Biology, 1988, ed. Ausubel et al., J. Wiley & Sons publ., New York, and Tijssen, 1993, Techniques in Biochemistry and Molecular BiologyHybridization with Nucleic Acid Probes, "Overview of principles of hybridization and the strategy of nucleic acid assays,". Generally, stringent conditions are selected to be about 5-30° C lower than the thermal melting point (Tm) for the specified sequence at a defined ionic strength and pH. Alternatively, stringent conditions are selected to be about 5-15° C lower than the thermal melting point (Tm) for the specified sequence at a defined ionic strength and pH. The Tm is the temperature (under defined ionic strength, pH and nucleic acid concentration) at which 50% of the probes complementary to the target hybridize to the target sequence at equilibrium (as the target sequences are present in excess, at Tm, 50% of the probes are occupied at equilibrium). For example, stringent hybridization conditions will be those in which the salt concentration is less than about 1.0 M sodium (or other salts) ion, typically about 0.01 to about 1 M sodium ion concentration at about pH 7.0 to about pH 8.3 and the temperature is at least about 25° C for short probes (e.g., 10 to 50 nucleotides) and at least about 55° C for long probes (e.g., greater than 50 nucleotides). Stringent conditions may also be modified with the addition of hybridization destabilizing agents such as formamide.

The terms "selective" or "selective conditions", as used herein, denote hybridization conditions for the primers and/or probes of the invention that permit amplification, detection and/or quantification of a target nucleic acid in a sample that may contain additional non-targetted nucleic acids.

The "complement" of a nucleic acid sequence, as used herein, refers to an oligonucleotide which, when aligned with the nucleic acid sequence such that the 5'-end of one sequence is paired with the 3'-end of the other, is in anti-parallel association. The complement of a nucleic acid sequence need not exactly match every nucleotide of the sequence; stable duplexes may contain mismatched base pairs or unmatched bases. Those skilled in the art of nucleic acid technology can determine duplex stability by empirically considering a number of variables including, for example, the length of the oligonucleotide, base composition and sequence of the oligonucleotide, ionic strength, and incidence of mismatched base pairs.

Stability of a nucleic acid duplex is measured by the melting temperature, or "Tₘ". The Tₘ of a particular nucleic acid duplex under specified conditions is the temperature at which half of the potential base pairs are disassociated.

As used herein, the term "probe" refers to an oligonucleotide which can form a duplex structure with a region of a target nucleic acid, due to complementarity of at least one sub-sequence in the probe with a sub-sequence in the target nucleic acid. The probe, preferably, does not contain a sequence complementary to sequence(s) of a primer. As discussed below, the probe can be labeled or unlabeled. The 3' terminus of the probe can be "blocked" to prohibit incorporation of the probe into a primer extension product. "Blocking" can be achieved by using non-complementary bases or by adding a chemical moiety such as biotin or a phosphate group to the 3' hydroxyl of the last nucleotide, which may, depending upon the selected moiety, serve a dual purpose by also acting as a label for subsequent detection or capture of the nucleic acid attached to the label. Blocking can also be achieved by removing the 3' hydroxyl or by using a nucleotide that lacks a 3' hydroxyl such as a dideoxynucleotide.

The phrase "the 5'-end of the probe" refers to that portion of an immobilized probe of the invention that is digested by an enzyme having 5'nuclease activity.

The phrase "the 3'-end of the probe" refers to that portion of an immobilized probe of the invention that remains attached to the solid substrate after exposure to an enzyme having 5'-nuclease activity.

The term "label moiety" refers to a compound that is releasable from an immobilized probe of the invention upon exposure to an enzyme having 5'-nuclease activity. A label moiety can be a fluorescent moiety or a quencher moiety, as described below. In certain embodiments, a label moiety can be a non-fluorescing detectable moiety, for example, a radioactive isotope *(e.g.,* ³H, ³²P, ¹²⁵I), a biotin or a biotin binding moiety (*e.g*., avidin, streptavidin, neutravidin, etc.).

The term "fluorescent moiety" or "fluorophore moiety" as used herein interchangeably refer to a chemical moiety that can emit light under conditions appropriate for the particular moiety. Typically, a particular fluorescent moiety or fluorophore moiety can emit light of a particular wavelength following absorbance of light of shorter wavelength. The wavelength of the light emitted by a particular fluorescent moiety is characteristic of that moiety. Thus, a particular fluorescent moiety can be detected by detecting light of an appropriate wavelength following excitation of the fluorescent moiety with light of shorter wavelength. Examples of fluorescent moieties that can be used in the methods and compositions of the present invention include, but are not limited to, fluorescein-family dyes, polyhalofluorescein-family dyes, hexachlorofluorescein-family dyes, coumarin-family dyes, rhodamine-family dyes, cyanine-family dyes, oxazine-family dyes, thiazine-family dyes, squaraine-family dyes, chelated lanthanide-family dyes, and BODIPY^{®}-family dyes.

The term "quencher moiety" as used herein refers to a chemical moiety that can absorb energy emitted by a fluorescent moiety when the quencher moiety is sufficiently close to the fluorescent moiety, for example, when both the quencher and fluorescent moiety are linked to a common polynucleotide. This phenomenon is generally known in the art as fluorescent resonance energy transfer ("FRET"). A quencher moiety can re-emit the energy absorbed from a fluorescent moiety in a signal characteristic for that quencher moiety, and thus a quencher can also be a "fluorescent moiety." Alternatively, a quencher moiety may dissipate the energy absorbed from a fluorescent moiety as heat or other non-radioactive process. Examples of quencher moieties that can be used in the methods and compositions of the present invention include, but are not limited to, fluorescein-family dyes, polyhalofluorescein-family dyes, hexachlorofluorescein-family dyes, coumarin-family dyes, rhodamine-family dyes, cyanine-family dyes, oxazine-family dyes, thiazine-family dyes, squaraine-family dyes, chelated lanthanide-family dyes, BODIPY^{®}-family dyes, azo-family dyes, triphenylmethane-family dyes, low-fluorescent quencher moieties (*i.e*., "dim donors") and non-fluorescent quencher moieties (*e.g*., so-called "dark quenchers" including Black Hole Quenchers™ (BHQ)).

As defined herein, "5'- to 3'-nuclease activity" or "5'-nuclease activity" interchangeably refer to that activity of an enzyme whereby nucleotides are removed from the 5'-end of an oligonucleotide in a sequential manner. The 5'-nuclease activity is preferably a 5'- to 3'-exonuclease activity, but can also be a 5'- to 3'-endonuclease activity in certain embodiments. For example, many template-specific nucleic acid polymerases exhibit a 5'- to 3'-exonuclease activity that is traditionally associated with some DNA polymerases, (i.e., *E*. *coli* DNA polymerase I has this activity whereas the Klenow fragment of *E. coli* DNA polymerase I does not). The 5'- to 3'-exonuclease activity can also cleave a substrate nucleic acid more than one phosphodiester bond (nucleotide) from the 5'-end of the substrate. Although not intending to be bound by any particular theory of operation, it is believed that this aspect of 5'- to 3'-exonuclease activity associated with DNA polymerases, which leads to release of cleaved oligonucleotide fragments from probes, can depend upon the particular nucleotide composition of the probe. For instance, the number of matches or mismatches between nucleotides of the oligonucleotide and template nucleic acid, particularly at the 5'-end of the oligonucleotide, can influence this activity, as described, for example, by Holland et al., 1991, Proc. Natl. Acad. Sci. USA 88:7276-80. 5'-Nuclease reactions are based on those described in U.S. Pat. Nos. 6,214,979, 5,804,375, 5,487,972 and 5,210,015.

The term "adjacent" in the context of probe and primer hybridization to a target nucleic acid refers to the positioning of the primer with respect to the probe on the same or the complementary strand of the template nucleic acid. The primer and probe may be separated by more than about 150 nucleotides, more than about 125 nucleotides, more than about 100 nucleotides, more than about 80 nucleotides, more than about 60 nucleotides, more than about 50 nucleotides, more than about 40 nucleotides, more than about 30 nucleotides, more than about 20 nucleotides, by about 1 to about 20 nucleotides, by about 1 to about 10 nucleotides, or may directly abut one another without overlapping. In certain embodiments, the probe is separated from the primer by about 1 to about 10 nucleotides. Generally, the probe can hybridize to the target nucleic acid anywhere within the sequence to be amplified that is downstream of a primer, thus allowing primer extension to position the polymerase so that the probe is fragmented.

The term "adjacent" in the context of a "fluorophore moiety immobilized adjacent to an immobilized probe," the term "adjacent" refers to the location of an immobilized fluorophore moiety. The fluorophore moiety immobilized adjacent to an immobilized probe is located sufficiently close to a quencher moiety attached to the 5'-end of an immobilized probe such that its fluorescence is masked. The fluorophore moiety immobilized adjacent to an immobilized probe can be attached, for example, to another nucleic acid attached to the solid substrate or to the solid substrate itself, typically via a linking moiety.

The term "thermostable nucleic acid polymerase" refers to an enzyme which is relatively stable to heat when compared, for example, to nucleotide polymerases from *E*. *coli* and which catalyzes the polymerization of nucleoside triphosphates. Generally, such enzymes are obtained from organisms considered by those in the art to be thermophilic organisms. Generally, the enzyme will initiate synthesis at the 3'-end of a primer annealed to the primer binding sequence, and will continue synthesis of a new strand toward the 5'-end of the template. If the polymerase possesses a 5'- to 3'-nuclease activity, it can hydrolyze intervening probes annealed to the template to release both labeled and unlabeled probe fragments, until polymerization terminates or all probe fragments dissociate from the nucleic acid to be detected. A representative thermostable enzyme isolated from *Thermus aquaticus* (Taq) is described in U.S. Pat. No. 4,889,818 and a method for using it in conventional PCR is described in Saiki et al., 1988, Science 239:487-91. Another representative thermostable enzyme includes *Thermus* species Z05 DNA polymerase. *See, e.g.,* U.S. Patent No. 5,674,738."

Taq DNA polymerase has a DNA synthesis-dependent, strand replacement 5'-3'-exonuclease activity (see Gelfand, "Taq DNA Polymerase" in PCR Technology Principles and Applications for DNA Amplification, Erlich, Ed., Stockton Press, N.Y. (1989), Chapter 2). Thus, Taq DNA polymerase does not degrade the probe when it is unbound to template DNA.

To determine "percent complementarity" or "percent identity" of two nucleic acid sequences, the sequences are aligned for optimal comparison purposes (e.g., gaps can be introduced in the sequence of a first nucleic acid sequence for optimal alignment with a second nucleic acid sequence). The nucleotides at corresponding nucleotide positions are then compared. When a position in the first sequence is occupied by a complementary nucleotide as the corresponding position in the second sequence, then the molecules are complementary at that position. Likewise, when a position in the first sequence is occupied by the same nucleotide as the corresponding position in the second sequence, then the molecules are identical at that position. The percent complementarity (or percent identity) between the two sequences is a function of the number of complementary positions (or identical positions) shared by the sequences divided by the total number of positions compared (i.e., % complementarity = number of complementary overlapping positions/total number of positions of the shorter nucleotide x 100%; and % identity = number of identical overlapping positions/total number of positions of the shorter nucleotide x 100%).

The determination of percent identity between two sequences can also be accomplished using a mathematical algorithm. A, non-limiting example of a mathematical algorithm utilized for the comparison of two sequences is the algorithm of Karlin and Altschul, 1990, Proc. Natl. Acad. Sci. U.S.A. 87:2264-2268, modified as in Karlin and Altschul, 1993, Proc. Natl. Acad. Sci. U.S.A. 90:5873-5877. Such an algorithm is incorporated into the NBLAST program of Altschul et al., 1990, J. Mol. Biol. 215:403.

The practice of the present invention will employ, unless otherwise indicated, conventional techniques of molecular biology, microbiology and recombinant DNA techniques, which are within the skill of the art. Such techniques are explained fully in the literature. *See, e.g.,* Sambrook et al., 2001, Molecular Cloning: A Laboratory Manual, Third Edition, Cold Spring Harbor Laboratory Press, Cold Spring Harbor, New York; Oligonucleotide Synthesis (M. J. Gait, ed., 1984); Nucleic Acid Hybridization (B. D. Hames & S. J. Higgins, eds., 1984); A Practical Guide to Molecular Cloning (B. Perbal, 1984); and a series, Methods in Enzymology (Academic Press, Inc.).

### "Plurality" refers to two or more.

### 4. Oligonucleotide Probes

In another aspect, the invention provides an immobilized probe comprising a releasable label moiety for the detection of a target nucleic acid. The probe can be any nucleic acid probe that can be used to identify the presence of a target nucleic acid known to one of skill in the art without limitation. Typically, the probe comprises a nucleotide sequence that hybridizes to a region in a target nucleic acid distinct from a region where a primer hybridizes. Typically, the probe is immobilized through its 3'-end to a solid substrate.

The probe nucleotide sequence can be of any length sufficient to specifically bind a target nucleic acid to be detected. In certain embodiments, the probe comprises at least about 6 nucleotides. In certain embodiments, the probe comprises fewer than about 140 nucleotides. In certain embodiments, the probe can be about 18 to about 45, about 25 to about 35, or about 25 to about 30 nucleotides in length. The length and composition of the probe can be chosen to give sufficient thermodynamic stability to ensure hybridization of the probe to the target nucleic acid under the appropriate reaction conditions, which depend on the detection method to be performed. The probes are typically designed to have a melting temperature (Tm) that is higher than the Tm of the primers. Probes with modified, non-standard, or derivatized nucleotides may be longer or shorter than those with conventional nucleotides while having similar thermodynamic hybridization properties. Examples of such non-standard bases may be found in U.S. Patent Nos. 6,320,005; 6,174,998; 6,001,611 and 5,990,303. As another example, probes with G/C-rich sequences may anneal to target sequences at higher temperatures that a probe of similar length with A/T-rich sequences.

Typically, the portion of the probe nucleotide sequence that hybridizes to the detectable nucleic acid is identical or complementary to the region of the detectable nucleic acid to which the probe hybridizes. However, this portion of the probe can have less than 100% sequence identity or complementarity to the region of the detectable target nucleic acid to which the probe hybridizes. In certain embodiments of the invention, nucleotide sequence of the portion of the probe that hybridizes to the detectable target nucleic acid can have about 99%, about 98%, about 97%, about 96%, about 95%, about 90%, about 85% or about 80% complementarity or identity to the region of the detectable target nucleic acid to which the probe hybridizes. The immobilized probes of the present invention are particularly suitable for genotyping, single-nucleotide polymorphism, and other base-pair mismatch analyses. Use of the immobilized, labeled probes allows for highly sensitive single-base discrimination in comparison to conventional linear arrays because of the cumulative destabilizing effect of the mismatched base pair and the internal label moiety.

The nucleic acid probes of the invention can additionally comprise other nucleic acid sequences that are not derived from and/or do not hybridize to a target nucleic acid. These additional nucleic acid sequences can be selected by one of skill in the art to provide desired functionality to the probes. For example, the nucleic acid probes can comprise additional sequences that allow improved methods of detection. Examples of probes that can comprise additional nucleic acid sequences or can otherwise be adapted for use in the probes, methods, and kits of the invention can be found in U.S. Patent Nos. 6,323,337; 6,248,526; 6,150,097; 6,117,635; 6,090,552; 5,866,336 and 5,723,591.

In addition to the probe nucleotide sequence, the probe can comprise additional nucleotide sequences or other moieties that do not inhibit the methods of the instant invention. In convenient embodiments of the invention, the probe can comprise additional nucleotide sequences that facilitate the methods of the invention. Moieties can be present within the probe that stabilize or destabilize hybridization of the probe or probe fragments with the nucleotide sequence. For example, an oligo T linker can be used to facilitate hybridization. The probes of the invention can also comprise modified, non-standard, or derivatized nucleotides as defined above.

The nucleic acid probes of the invention can be prepared by any method known to one of skill in the art without limitation. In particular, the methods used to prepare the nucleic acid primers of the invention described above may also be used to prepare the nucleic acid probes of the invention.

One or more label moieties can be attached to the probe directly or indirectly by a variety of techniques. In some embodiments, the label moiety is located at the 5'-end of the probe (i.e., the-end of the probe that is digested by the enzyme having 5'-nuclease activity), located internally in the probe's nucleotide sequence, or attached to spacer arms of various sizes and compositions to facilitate signal interactions. Typically, a label moiety is attached via a non-nucleosidic linker, rather than attached to a nucleoside base. Using commercially available phosphoramidite reagents, one can produce oligonucleotides containing functional groups (e.g., thiols or primary amines) at either terminus via an appropriately protected phosphoramidite, and can attach a label moiety thereto using protocols described in, for example, PCR Protocols: A Guide to Methods and Applications, ed. by Innis et al., Academic Press, Inc., 1990.

Methods for introducing oligonucleotide functionalizing reagents to introduce one or more sulfhydryl, amino or hydroxyl moieties into the oligonucleotide probe sequence, typically at the 5' terminus are described in U.S. Pat. No. 4,914,210. Other methods of attaching a label moiety, including a fluorescent moiety, a quencher moiety or a non-fluorescing detectable moiety, to the probe can be found in U.S. Patent No. 5,118,802. The method of linking or conjugating the label moiety to the probe depends, of course, on the type of label moiety or moieties used and the position of the label moiety on the probe.

In certain embodiments, the one or more label moieties can be attached to the 5'-end of the probe. In other embodiments, the one or more label moieties can be attached to the probe at a residue that is internal to the 5'-end of the probe, for example, within 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, about 35, or about 40 residues from the 5' terminal position of the probe. In certain embodiments, the one or more label moieties can be attached to the 3'-end of the probe. In other embodiments, the one or more label moieties can be attached to the probe at a residue that is internal to the 5'-end of the probe, for example, within 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, about 35, or about 40 residues from the 3' terminal position of the probe. The one or more label moieties can be attached to any portion of a residue of the probe. For example, the one or more label moieties can be attached to a sugar, phosphate, or base moiety of a nucleotide in the probe. In other embodiments, the one or more label moieties can be attached between two residues of the probe.

In certain embodiments of the invention, a probe can be labeled with more than one label moiety. In certain of such embodiments, each label moiety can be individually attached to different bases of the probe. In other embodiments, more than one label moiety can be attached to the same base of the probe.

In certain embodiments, the immobilized probe comprises a fluorescent moiety. The fluorescent moiety can be any fluorescent moiety known to one of skill in the art without limitation. In general, fluorescent moieties with wide Stokes shifts are preferred, allowing the use of fluorometers with filters rather than monochromometers and increasing the efficiency of detection. In certain embodiments, the fluorescent moiety can be selected from the group consisting of fluorescein-family dyes (Integrated DNA Technologies, Inc., Coralville, IA), polyhalofluorescein-family dyes (ABI, Foster City, CA), hexachlorofluorescein-family dyes (ABI, Foster City, CA), coumarin-family dyes (Invitrogen-Molecular Probes, Inc., Eugene, OR), rhodamine-family dyes (GE Healthcare), cyanine-family dyes, oxazine-family dyes, thiazine-family dyes, squaraine-family dyes, chelated lanthanide-family dyes, and BODIPY^{®}-family dyes (Molecular Probes, Inc.). In a embodiment, the fluorescent moiety is 6-carboxyfluorescein (FAM™). Other examples of fluorescent moieties that can be used in the probes, methods, and kits of the invention can be found in U.S. Patent Nos. 6,406,297; 6,221,604; 5,994,063; 5,808,044; 5,880,287; 5,556,959 and 5,135,717. Fluorescent moieties of use in the present invention are commercially available from, for example, Invitrogen-Molecular Probes, Eugene, OR.

In certain embodiments, the immobilized probe comprises a quencher moiety. The quencher moiety can be selected from the group consisting of fluorescein-family dyes, polyhalofluorescein-family dyes, hexachlorofluorescein-family dyes, coumarin-family dyes, rhodamine-family dyes, cyanine-family dyes, oxazine-family dyes, thiazine-family dyes, squaraine-family dyes, chelated lanthanide-family dyes, BODIPY^{®}-family dyes, low-fluorescent quencher moieties and non-fluorescent quencher moieties. In certain embodiments, the non-fluorescent quencher moieties can be BHQ™-family dyes (including the quenchers described in WO 01/86001, BHQ-1, BHQ-2, BHQ-3) (BioSearch Technologies, Novato, CA), Iowa Black™, or Dabcyl (Integrated DNA Technologies, Inc.). Other examples of specific quencher moieties include, for example, but not by way of limitation, TAMRA (N,N,N',N'-tetramethyl-6-carboxyrhodamine) (Invitrogen-Molecular Probes, Inc.), DABCYL (4-(4'-dimehtylaminophenylazo)benzoic acid), Iowa Black™ (Integrated DNA Technologies, Inc.), Cy3™ (GE Healthcare) or Cy5™ (GE Healthcare). In one embodiment, the quencher moiety is BHQ-2. Other examples of quencher moieties that can be used in the probes, methods, and kits of the invention can be found in U.S. Patent Nos. 6,399,392, 6,348,596, 6,080,068, and 5,707,813. Quencher moieties of use in the present invention are commercially available from, for example, Molecular Probes, Eugene, OR.

In certain embodiments of the invention, the probe can comprise a fluorescent moiety and a quencher moiety. In such embodiments, the fluorescent moiety can be any fluorescent moiety known to one of skill in the art, as described above. Further, the quencher moiety can be any quencher moiety known to one of skill in the art without limitation. Typically, the quencher moiety will be attached to the 5'-end of the probe and the fluorescent moiety will be attached to the probe 3' from where the quencher moiety is attached.

While not intending to be bound to any particular theory or mechanism of action, it is believed that when the probe is intact, a photon emitted by the fluorescent moiety can be absorbed and thus quenched by the quencher moiety. The quencher moiety then either releases the energy of the photon as a photon of different wavelength or as heat. Thus, the quencher moiety can also be a fluorescent moiety. As described above, this phenomenon is termed fluorescence resonance energy transfer ("FRET"). Cleaving the probe between the fluorescent moiety and quencher results in a reduction in quenching of the fluorescent moiety's emitted fluorescence by the quencher moiety.

Generally, transfer of energy between the fluorescent moiety and the quencher moiety depends on the distance between the fluorescent moiety and the quencher moiety and the critical transfer distance of the particular fluorescent moiety-quencher moiety pair. The critical transfer distance is both characteristic and constant for a given fluorescent moiety paired with a given quencher moiety. Further, the spatial relationship of the fluorescent moiety in reference to the quencher moiety can be more sensitively determined when the critical transfer distance of the fluorescent moiety-quencher moiety pair is close to the distance between the fluorescent moiety and the quencher moiety. Accordingly, the skilled practitioner can select the fluorescent moiety and the quencher moiety to have a critical transfer distance that is close to the distance separating the fluorescent moiety from the quencher moiety on the probe. Critical transfer distances of particular fluorescent moiety-quencher moiety pairs are well known in the art and can be found, for example, in an article by Wu and Brand, 1994, Anal. Biochem. 218:1-13.

Other criteria for section of particular fluorescent moiety-quencher moiety pairs include, for example, the quantum yield of fluorescent emission by the fluorescent moiety; the wavelength of fluorescence emitted by the fluorescent moiety; the extinction coefficient of the quencher moiety; the wavelength of fluorescence, if any, emitted by the quencher moiety; and the quantum yield of fluorescent emission, if any, by the quencher moiety. In addition, if the quencher moiety is also a fluorescent moiety, the quencher moiety and the fluorescent moiety can preferably be selected so that fluorescence emitted by one can easily be distinguished from fluorescence emitted by the other.

Further guidance on the selection of particular fluorescent moiety-quencher moiety pairs may be found in a review article by Klostermeier and Millar, 2002, Biopolymers 61:159-179.

Exemplary combinations of fluorescent moieties and quencher moieties that can be used in the present invention include, but are not limited to, a 6-carboxyfluorescein (FAM) fluorophore moiety and a Cy5™ quencher moiety; a fluorophore moiety selected from the group consisting of FAM, TET, JOE, HEX, Oregon Green and a BHQ-1 quencher moiety; a fluorophore moiety selected from the group consisting of FAM, TAMRA, ROX, Cy3, Cy3.5, CAL Red, Red 640 and a BHQ-2 quencher moiety; a fluorophore moiety selected from the group consisting of Cy5 and Cy5.5 and a BHQ-3 quencher moiety. Other examples of fluorescent moiety-quencher moiety pairs that can be used in the probes, methods, and kits of the invention can be found in U.S. Patent No. 6,245,514.

Examples of molecules that can be used as both fluorescent or quencher moieties in FRET include fluorescein, 6-carboxyfluorescein, 2'7'-dimethoxy-4'5'-dichloro-6-carboxyfluorescein, rhodamine, 6-carboxyrhodamine, 6-carboxy-X-rhodamine, and 5-(2'-aminoethyl)aminonaphthalene-1-sulfonic acid (EDANS). Whether a fluorescent moiety is a donor or an acceptor is defined by its excitation and emission spectra, and the fluorescent moiety with which it is paired. For example, FAM™ is most efficiently excited by light with a wavelength of 494 nm, and emits light with a spectrum of 500 to 650 nm, and an emission maximum of 525 nm. Accordingly, FAM™ is a suitable fluorescent moiety for use with, for example, TAMRA as quencher moiety, which has at its excitation maximum 555 nm.

In certain embodiments, the label moiety is a non-fluorescing detectable moiety, for example, a radioactive isotope, a biotin moiety or a biotin binding moiety (*e.g*., avidin, streptavidin, neutravidin). When the non-fluorescing detectable moiety is a biotin or biotin binding moiety, any detectable label known in the art attached to a secondary biotin binding moiety or a biotin, respectively, can be used to detect a target nucleic acid. For example, a radioisotope, an enzyme (*e.g*., alkaline phophatase, horseradish peroxidase), or a fluorophore can be attached to a secondary biotin binding moiety or a biotin.

The probes are typically linked to a solid substrate via its 3' end. Typically, the probes are covalently bound, but can also be non-covalently attached to the substrate. The particular linkage between an immobilized probe and a solid substrate is not critical to the success of the invention so long as the linkage can withstand conditions required for an amplification reaction. For example, in a PCR reaction, the linkage should withstand intermittent and repeated exposure to temperatures for denaturing nucleic acid sequences (80°C-105°C, usually about 95°C). In some embodiments the probe can be attached to the solid substrate via a residue that is within 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, about 35, or about 40 residues from the 3' terminal position of the probe. Typically, the probe will be attached to the solid substrate through its 3' terminus. The probe can be modified to contain a nucleophile group *(i.e.,* an amino group) or an electrophile group on its 3' end. The probes generally can be modified using phosphoramidite chemistry methods known in the art. For example, nucleoside amino-modifier phosphoramidites are commercially available from Glen Research of Sterling, Virginia. Alternatively, the probes can be linked to activated chemical groups on solid substrates that bind to oligonucleotides, for example, a carboxylate, an aldehyde moiety, an epoxide moiety, an NHS ester including an N-hydroxysuccinimide moiety, an amino moiety, a nitro moiety, a hydroxyl moiety, an isothiocyanate moiety, a dichlorotriazonyl moiety. Typical activated chemical groups include an epoxide moiety, a carboxylate moiety, an NHS ester, and an aldehyde moiety. Solid substrates comprising activated chemical moieties for use in the capture of oligonucleotides are commercially available from Telechem International, Inc., Sunnyvale, CA, Nalge Nunc, Intl., Rochester NY and Exiqon, Vedbaek, Denmark. Additional linking moieties useful for immobilizing the probes of the present invention are commercially available from Pierce Biotechnology, Rockford, IL.

### 5. Solid Substrates

The solid substrates can composed of any material able to withstand conditions required for the amplification reaction. For example, in PCR the solid substrate should withstand intermittent and repeated exposure to temperatures for denaturing nucleic acid sequences (80°C-105°C, usually about 95°C). Exemplified materials for composition of the solid substrates include without limitation glass, a silicon composition, plastic, synthetic polymer (*i.e*., polypropylene, polystyrene, polycarbonate, polyvinyl, etc.), metal, ceramic, etc. The solid substrate will typically have a flat surface on which to attach the oligonucleotide probes, and can be any shape appropriate to a particular assay or apparatus for carrying out the present methods. Exemplified shapes for the solid substrates include circles, ovals, squares, rectangles, triangles, and any other polygonal shape. The solid substrate can take the form of a flat-surfaced disk, a multi-well plate, an assay tube *(i.e.,* a PCR tube), a microscope slide. A flat-surfaced disk for use in the present methods will typically have a surface area of about 1.0 mm² to 1.0 cm², for example, about 1.0 mm², 2.0 mm², 3.0 mm², 4.0 mm², 5.0 mm², 6.0 mm², 7.0 mm², 8.0 mm², 9.0 mm², 1.0 cm², 2.0 cm², 3.0 cm², 4.0 cm², 5.0 cm². The flat-surfaced disks of the invention have a thickness of about 0.5 mm to about 1.0 cm, more usually about 1.0 mm, 2.0 mm, 3.0 mm, 4.0 mm, 5.0 mm, 6.0 mm, 7.0 mm, 8.0 mm, 9.0 mm. Microwell plates appropriate for use in the present methods include those commercially available from, for example, Corning Lifesciences, Acton, MA (DNA binding Thermowell^{®} plates) and Nalge Nunc, Intl. (ArrayCote™ plates). Microscope slides of use in the present invention include those available from, for example, Telechem International, Inc., Sunnyvale, CA (*i.e*., SuperEpoxide and SuperAldehyde slides), Asper Biotech, Tartu, Estonia (*i.e*., 3-aminopropyltrimethoxysilane + 1,4-phenylenediisothiocyanate activated slides), Nalge Nunc, Intl., Rochester NY (*i.e*., Nucleolink™ microarray slides), Grace Bio-Labs, Bend, OR (*i.e*., Hybriwell chamber slides), and Bio-Rad Laboratories, South San Francisco, CA (*i.e*., Frame-Seal system).

Flat-surfaced solid substrates typically will have a reaction chamber, for example, a sealable depression well or a sealable raised reaction vessel to contain amplification reagents for an amplification reaction. The depression well or reaction vessel should seal such that evaporation of the intended volume of fluid for the amplification reaction does not occur. Preferably, a raised reaction vessel is optionally removable from the flat-surfaced solid substrate. The reaction chambers can be sealed, for example, with a tight-fitting cover or with a rubber or silicon gasket ring. Leakage or out-gassing can be minimized or prevented by sealing the closure with glue or caulking, or by maintaining the reaction chamber under pressure.

In some embodiments, the solid substrate is treated with a blocking agent (i.e., bovine serum albumin (BSA)) to minimize or prevent absorption of critical enzymes or components.

### 6. Methods for Detecting and/or Quantifying a Target Nucleic Acid

Generally, the methods comprise detecting a target nucleic acid by amplifying the target nucleic acid in the presence of an oligonucleotide probe immobilized to a solid substrate via its 3'end and comprising a label moiety at its 5'-end. These methods generally comprise contacting an immobilized probe hybridized to a target nucleic acid with an enzyme having 5'-nuclease activity in the presence of amplification reagents. The enzyme with 5'-nuclease activity then fragments the immobilized probe hybridized to the target nucleic acid in a 5'-nuclease reaction, thereby releasing the label moiety attached to the 5'-end. The probe can be labeled with a label moiety that enables detection of fragmentation of the probe. Such methods are based on those described in U.S. Pat. Nos. 6,214,979, 5,804,375, 5,487,972 and 5,210,015.

In a 5'-nuclease reaction, the nucleic acid, primer and immobilized probe can be contacted with any enzyme known by one of skill in the art to have 5'- to 3'-nuclease activity without limitation. The conditions are preferably chosen to permit the enzyme to cleave the 5'-end of the immobilized probe and release a plurality of fragments of the probe from the nucleic acid. Preferred enzymes with 5'-nuclease activity include template-dependent nucleic acid polymerases. Known native and recombinant forms of such polymerases include, for example, *E*. *coli* DNA polymerase I (Fermentas, Inc., Hanover, MD), *Bacillus stearothermophilus* DNA polymerase, and *Thermococcus littoralis* DNA polymerase.

In preferred embodiments, the enzymes with 5'-nuclease activity are thermostable and thermoactive nucleic acid polymerases. Such thermostable polymerases include, but are not limited to, native and recombinant forms of polymerases from a variety of species of the eubacterial genera *Thermus, Thermatoga,* and *Thermosipho, ,* as well as chimeric forms thereof. For example, *Thermus* species polymerases that can be used in the methods of the invention include *Thermus aquaticus* (Taq) DNA polymerase, *Thermus thermophilus* (Tth) DNA polymerase, Thermus species Z05 (Z05) DNA polymerase, and *Thermus* species sps17 (sps17), as described in U.S. Pat. Nos. 5,405,774; 5,352,600; 5,079,352; 4,889,818; 5,466,591; 5,618,711; 5,674,738, and 5,795,762. *Thermatoga* polymerases that can be used in the methods of the invention include, for example, *Thermatoga maritima* DNA polymerase and *Thermatoga neapolitana* DNA polymerase, while an example of a *Thermosipho* polymerase that can be used is *Thermosipho africanus* DNA polymerase. The sequences of *Thermatoga maritima* and *Thermosipho africanus* DNA polymerases are published in International Patent Application No. PCT/US91/07035 with Publication No. WO 92/06200. The sequence of *Thermatoga neapolitana* may be found in International Patent Publication No. WO 97/09451.

A 5'-nuclease reaction comprises contacting the target nucleic acid to be detected with a primer, an immobilized probe of the invention, and an enzyme having 5'- to 3'-nuclease activity, under conditions in which the primer and the immobilized probe hybridize to the nucleic acid. Components of a 5'-nuclease reaction can contact the nucleic acid to be detected in any order, e.g., the primer can contact the nucleic acid to be detected first, followed by the immobilized probe and enzyme with 5'-nuclease activity, or alternatively the enzyme with 5'-nuclease activity can contact the nucleic acid to be detected first, followed by the immobilized probe and primer. In certain embodiments, more than one primer or immobilized probe may be added to a 5'-nuclease reaction. In certain preferred embodiments, a pair of primers can contact the nucleic acid in a 5'-nuclease reaction. The primer can be any primer capable of priming a DNA synthesis reaction. Where only one primer is used, the primer should hybridize to the target nucleic acid upstream of the immobilized probe, *i.e*., the 3'-end of the primer should point toward the 5'-end of the immobilized probe. The 3'-end of the primer can hybridize adjacent to the 5'-end of the probe or adjacent to the 3'-end of the primer can hybridize further upstream of the 5'-end of the probe. Where more than one primer is used, at least one primer should hybridize to the nucleic acid to be detected upstream of the immobilized probe, as described above.

Certain embodiments of the 5'-nuclease reactions of the present invention are based on several 5'-nuclease reactions that are known to those of skill in the art. Examples of such reactions are described in detail, for instance, in U.S. Pat. No. 5,210,015.

Briefly, in a 5'-nuclease reaction, a target nucleic acid is contacted with a primer and an immobilized probe under conditions in which the primer and immobilized probe hybridize to a strand of the target nucleic acid. The nucleic acid, primer and immobilized probe are also contacted with an enzyme, for example a polymerase, having 5'- to 3'-nuclease activity. Nucleic acid polymerases possessing 5'- to 3'-nuclease activity can cleave the immobilized probe hybridized to the target nucleic acid downstream of the primer. The 3'-end of the primer provides a substrate for extension of a new nucleic acid as based upon the template nucleic acid by the nucleic acid polymerase. As the polymerase extends the new nucleic acid, it encounters the 5'-end of the probe and begins to cleave fragments from the immobilized probe.

In one embodiment, the primer and immobilized probe can be designed such that they hybridize to the target nucleic acid in close proximity to each other such that binding of the nucleic acid polymerase to the 3'-end of the primer puts it in contact with the 5'-end of the probe (*i.e*., adjacent without overlapping or abutting).

Alternatively, if the primer and immobilized probe anneal to more distantly spaced regions of the nucleic acid, nucleic acid extension must occur before the nucleic acid polymerase encounters the 5'-end of the probe. As the polymerization continues, the polymerase progressively cleaves fragments from the 5'-end of the probe. This cleaving continues until the remainder of the probe has been destabilized to the extent that it dissociates from the template molecule. The term "polymerization-dependent cleavage" refers to this process.

Regardless of the region of a target nucleic acid hybridized by an immobilized probe, a sample is provided which contains the target nucleic acid. If the nucleic acid is double-stranded, it should first be denatured, e.g., the strands of the nucleic acid separated from each other. Any suitable denaturing method, including physical, chemical, or enzymatic means, known to one of skill in the art without limitation can be used to separate the nucleic acid strands. A preferred physical means for strand separation is heating the nucleic acid until it is completely (>99%) denatured. Typical heat denaturation involves temperatures ranging from about 80 °C to about 105 °C, for about 10 seconds to about 10 minutes. As an alternative to denaturation, the nucleic acid may exist in a single-stranded form in the sample, such as, for example, single stranded RNA or DNA viruses.

If the target nucleic acid to be detected is RNA, the RNA can either be used as an RNA template for a 5'-nuclease reaction as described above, or the RNA can be used as a template for reverse-transcription into cDNA, or both simultaneously or sequentially.
In certain embodiments, the RNA can be detected without reverse-transcription into cDNA using the methods of the invention. In other embodiments, the RNA can be first reverse-transcribed into cDNA in the absence of an immobilized probe, and then the cDNA product can be detected according to the methods of the invention. In still other embodiments, the RNA can be reverse-transcribed in the presence of an immobilized probe, simultaneously producing cDNA that can subsequently be amplified and/or detected and detecting the presence of the RNA by assessing fragmentation of the immobilized probe as described herein.

Where the RNA is reverse-transcribed in the absence of an immobilized probe, the RNA can be reverse transcribed into cDNA by any method known to one of skill in the art. The products of such reverse transcription can then be detected like any detectable nucleic acid according to the methods described herein.

Where the RNA is reverse-transcribed in the presence of an immobilized probe, the RNA can be reverse-transcribed by a DNA polymerase that can use RNA as a template for DNA strand synthesis. As with all known DNA polymerase synthesis activities, such synthesis requires the presence of a primer, such as those described herein. The DNA polymerase that can use RNA as a template is preferably thermostable, so that multiple cycles of denaturation and DNA synthesis can occur without destroying the polymerase. Further, the DNA polymerase used for reverse transcription can preferably also synthesize DNA using a DNA template. Such polymerases are described in, for example, U.S. Patent Nos. 6,468,775 *(Carboxydothermus hydrogenformans* DNA polymerase), 5,968,799 *(Thermosipho africanus* DNA polymerase), 5,736,373 *(Bacillus pallidus* DNA polymerase), 5,674,738 *(Thermus* species Z05 DNA polymerase), and 5,407,800 *(Thermus aquaticus* and *Thermus thermophilus* DNA polymerases). In addition, methods and compositions for reverse transcribing an RNA using a thermostable DNA polymerase with reverse transcription activity are described in U.S. Patent Nos. 5,693,517, 5,561,058, 5,405,774, 5,352,600, 5,310,652, and 5,079,352.

Whether RNA or DNA, the denatured nucleic acid strand is then contacted with a primer and an immobilized probe under hybridization conditions, which enable the primer and immobilized probe to bind to the nucleic acid strand. In certain embodiments, two primers can be used to amplify the nucleic acid. In such embodiments, the two primers can be selected so that their relative positions along the nucleic acid are such that an extension product synthesized from one primer, after the extension produce is separated from its template (complement), can serve as a template for the extension of the other primer to yield an amplified product of defined length. The length of the product depends on the length of the sequence between the two primers and the length of the two primers themselves.

Because the complementary strands are typically longer than either the probe or primer, the strands have more points of contact and thus a greater chance of finding and binding each other over any given period of time. A high molar excess of immobilized probe and primer helps shift the equilibrium toward primer and probe annealing to one strand of the target nucleic acid rather than both strands of the target nucleic acid reannealing.

The primer should be sufficiently long to prime the synthesis of extension products in the presence of the agent for polymerization. The exact length and composition of the primer can depend on many factors, including temperature of the annealing reaction, source and composition of the primer, proximity of the immobilized probe annealing site to the primer annealing site, and ratio of primer:probe concentration. For example, depending on the complexity of the sequence, an oligonucleotide primer typically contains about 15-30 nucleotides, although it may contain fewer or more nucleotides. The primers must be sufficiently complementary to selectively anneal to their respective strands and form stable duplexes.

Each primer can be selected to be "substantially" complementary to a strand of the nucleic acid. The primers need not reflect the exact sequence of the template, but must be sufficiently complementary to selectively hybridize to their respective strands under the appropriate reaction conditions. Non complementary bases or longer sequences can be interspersed into the primer or located at the ends of the primer, provided the primer retains sufficient complementarity with its template strand to form a stable duplex therewith. The non-complementary nucleotide sequences of the primers may include restriction enzyme sites. Any non-complementary nucleotide sequences are preferably not at the 3'-end of the primer.

The immobilized probe preferably hybridizes to the nucleic acid to be detected before the polymerase binds the nucleic acid and primer and begins to extend the new nucleic acid strand from the primer based upon the template of the detectable nucleic acid. It is possible for the polymerase to bind the primer and nucleic acid to be detected before the immobilized probe contacts the detectable nucleic acid; however, this arrangement can result in decreased immobilized probe fragmentation unless multiple cycles of primer extension are performed, as in a preferred PCR based 5'-nuclease reaction as described below. Accordingly, it is preferable that the immobilized probe hybridize to the nucleic acid to be detected before primer extension by the polymerase begins.

A variety of techniques known to one of skill in the art can be employed to enhance the likelihood that the immobilized probe will hybridize to the detectable nucleic acid before primer extension polymerization reaches this duplex region, or before the polymerase attaches to the upstream oligonucleotide. For example, short primer molecules generally require cooler temperature to form sufficiently stable hybrid complexes with the nucleic acid. Therefore, the probe can be designed to be longer than the primer so that the immobilized probe anneals preferentially to the nucleic acid at higher temperatures relative to primer annealing.

One can also use primers and immobilized probes having differential hybrid or duplex stability based upon their nucleotide composition. For example, the immobilized probe can be chosen to have greater G/C content and, consequently, greater duplex stability than the primer. Alternatively or additionally, one or more modified, non-standard or derivatized DNA bases may be incorporated into primers or probes to result in either greater or lesser duplex stability in comparison to primers or probes having only conventional DNA bases. Examples of such modified, non-standard or derivatized bases may be found in U.S. Patent Nos. 6,320,005, 6,174,998, 6,001,611, and 5,990,303.

Further, the temperature of the reaction can also be varied to take advantage of the differential hybrid or duplex stability of the probe and primer. For example, following denaturation at high temperatures as described above, the reaction can be incubated at an intermediate temperature which permits probe but not primer binding, followed by a further temperature reduction to permit primer annealing and subsequent extension.

The immobilized probes will typically be attached to a solid substrate at evenly spaced intervals, for example, in a grid or other regular or repeated pattern. Immobilized probes can be spaced from about 20 Å to about 1000 Å apart from one another, more usually from about 32 Å to about 700 Å apart.

Template-dependent extension of the oligonucleotide primer(s) is catalyzed by a DNA polymerase in the presence of adequate amounts of the four deoxyribonucleoside triphosphates (dATP, dGTP, dCTP, and TTP) or analogs, e.g., dUTP, as discussed above, in a reaction medium which is comprised of the appropriate salts, metal cations, and pH buffering system. Suitable polymerizing agents are enzymes known to catalyze primer and template-dependent DNA synthesis and can possess the 5'- to 3'-nuclease activity. Such enzymes include, for example, *Escherichia coli* DNA polymerase I, *Thermus thermophilus* DNA polymerase, *Bacillus stearothermophilus* DNA polymerase, *Thermococcus littoralis* DNA polymerase, *Thermus aquaticus* DNA polymerase, *Thermatoga maritima* DNA polymerase and *Thermatoga neapolitana* DNA polymerase and Z05 DNA polymerase. Further, the reaction conditions for performing DNA synthesis using these DNA polymerases are well known in the art. To be useful in the of the present invention, the polymerizing agent should possess 5'-nuclease activity that can efficiently cleave the immobilized oligonucleotide probe and release labeled fragments so that a detectable signal is directly or indirectly generated.

The products of the synthesis are duplex molecules consisting of the template strands and the primer extension strands. Byproducts of this synthesis are probe fragments which can consist of a mixture of mono-, di- and oligo- nucleotide fragments. In preferred embodiments, repeated cycles of denaturation, probe and primer annealing, and primer extension and cleavage of the probe can be performed, resulting in exponential accumulation of the amplified region defined by the primers and proportional generation of labeled fragments. Such repeated thermal cycling is generally known in the art as the polymerase chain reaction (PCR). Sufficient cycles can be performed to fragment a sufficient amount of the probe to distinguish positive reactions, i.e., the nucleic acid to be detected is present, from negative reactions, i.e., the nucleic acid to be detected is not present. Generally, positive reactions will exhibit a signal that is several orders of magnitude greater than a negative reaction.

In certain preferred embodiments, the PCR reaction is carried out as an automated process which utilizes a thermostable enzyme. In this process the reaction mixture is cycled through a denaturing step, a probe and primer annealing step, and a synthesis step, whereby cleavage and displacement occur simultaneously with primer dependent template extension. A thermal cycler, such as an ABI 3700 or an ABI 7700 (Applied Biosystems, Inc., Foster City, CA), which is specifically designed for use with a thermostable enzyme, may be employed. In certain of such embodiments of the invention, the nucleic acids to be detected can be amplified in the absence of a detectably-labeled probe, followed by detection of the amplification product in a separate reaction. Alternatively, the nucleic acids to be detected can be amplified in the presence of the probe, allowing amplification and detection in a single reaction.

Temperature stable polymerases are preferred in this automated process because the preferred way of denaturing the double stranded extension products is by exposing them to a high temperature (about 95 °C) during the PCR cycle. In certain embodiments of the present invention, it may be preferable to denature double-stranded DNA at lower temperatures, about 80-94 °C, for example, about 80 °C, 81 °C, 82°C, 83 °C, 84 °C, 85 °C, 86 °C, 87 °C, 88 °C, 89 °C, 90 °C, 91 °C, 92 °C, 93 °C or 94 °C in order to improve the stability of the linkage between the immobilized probe and solid substrate.

U.S. Pat. No. 4,889,818 discloses a representative thermostable enzyme isolated from *Thermus aquaticus.* Additional representative temperature stable polymerases include, e.g., polymerases extracted from the thermostable bacteria *Thermus flavus, Thermus ruber, Thermus thermophilus, Bacillus stearothermophilus* (which has a somewhat lower temperature optimum than the others listed), *Thermus lacteus, Thermus rubens, Thermotoga maritima, Thermococcus littoralis, Methanothermus fervidus,* and *Pyrococcus furiosus* (Stratagene, La Jolla, CA). As described above, certain of these thermostable polymerases can synthesize DNA from an RNA template. Where an RNA molecule is to be detected according to the methods of the invention, a DNA polymerase that can synthesize DNA from an RNA template, i.e., with reverse transcription activity, should be used, with any needed co-factors.

In other aspects, the methods of the present invention can also be used to quantify an amount of a target nucleic acid in a sample. In such methods, a 5'-nuclease reaction as described above is performed, and the amount of fluorescence produced is quantified. The amount of fluorescence can be quantified by any method known to one of skill in the art without limitation. In certain embodiments, the amount of fluorescence emitted can be quantified with a fluorometer. This amount of fluorescence can be compared to the amount of fluorescence emitted by a control reaction. The control reaction is preferably performed with the same reagents and at the same time as the reaction performed with the sample with a known amount of a nucleic acid having a known sequence. Alternatively, the amount of fluorescence emitted by the fluorescent moiety can be compared to a standard curve plotting fluorescence against a control target nucleic acid concentration. Further guidance in quantifying an amount of a nucleic acid of a member of the can be found, for example, in published U.S. Patent Application Publication No. 2002/0058262 and European Patent Nos. 1 138 780, 1 138 783, and 1 138 784.

In one embodiment of the invention, amplification in the presence of the immobilized probe is minimized. This method is particularly suited to an-end point determination of the presence or absence of a target nucleic acid, for example, for the purpose of genotyping or diagnosing a disease condition. The method involves first amplifying the target nucleic acid in the absence of an immobilized probe comprising a label moiety and then detecting the amplified target nucleic acid in the presence of the immobilized probe and an enzyme having 5'-nuclease activity. After exposure to the immobilized probe and enzyme having 5'-nuclease activity, the target nucleic acid is amplified through a minimal number of denaturation-annealing-extension cycles, usually about 10 cycles or less, for example, about 1, 2, 3, 4, 5, 6, 7, 8, 9, or 10 cycles.

An amplified target nucleic acid is typically detected by detecting a fluorescent signal. A fluorescent signal can be detected or monitored in "real-time" using a fluorescence reader with a programmed temperature control (*e.g*., PE/ABI 7700 Applied Biosystems), a photomultiplier tube, or a camera. Real-time detection finds use in quantitative analyses of a target nucleic acid in a sample. An end-point fluorescence signal can be detected subsequent to an amplification reaction using a spectrofluorometer (for example, directed to individual samples, a multiwell-plate or a flat-surfaced solid substrate, including a microscope slide). End-point detection finds use in qualitative analyses of a target nucleic acid in a sample, for example, in genotyping or diagnostic applications, particularly in detecting the presence or absence of a disease. The presence of released 5'-label in the reaction solution can be verified by capillary electrophoresis. The presence of amplicon can be correlated or verified by gel electrophoresis. Immobilized probe can be selectively cleaved or released from the solid substrate using an appropriate chemical (i.e., acid or base hydrolysis), enzymatic or physical reaction in conjunction with an appropriate cleavable linker moiety.

### 7. Apparatus for Practicing the Methods

The invention also contemplates an apparatus comprising a solid substrate attached to an immobilized oligonucleotide probe of the invention. The apparatus can be employed to carry out the present methods to detect the amplification of a single or multiple target nucleic acid sequences. The apparatus can allow for the simultaneous high throughput detection of multiple target nucleic acid sequences, for example as many as 6, 12, 24, 48, 96, 192, 384, 768, 1536 target nucleic acid sequences or more. In one embodiment, the apparatus comprises a chamber to accommodate the solid substrate, a fluid inlet port and a fluid outlet port, a vacuum and/or a pump to direct the flow of fluid *(i.e.,* amplification reagents), a thermocycling capability (*i.e*., a heating and/or a cooling device), and a fluorescence detector. In one embodiment, the apparatus is a microfluidic device utilizing microfluidics technology. Preferably, the chamber seals or is under pressure to prevent leakage and/or evaporation of a reaction mixture throughout nucleic acid amplification thermocycling. Exemplified microfluidics apparatus of use in carrying out the present methods include those commercially available from Caliper Life Sciences, Hopkinton, MA; Aclara Biosciences, Mountain View, CA; Fluidigm, South San Francisco, CA; and the Industrial Technology Research Institute, Hsinchu, Taiwan. Microfluidic systems and devices of use in practicing the present methods are described, for example, in U.S. Patent Nos. 6,881,312; 6,743,636; 6,673,593; 6,670,153; 6,630,353; 6,623,860; 6,541,274; 6,534,009; 6,524,830; 6,007,690.

### 8. Reaction Mixtures

In another aspect, the present invention provides reaction mixtures comprising an probe comprising a releasable label moiety immobilized to a solid substrate, as described above, and reagents for an amplification reaction, including a polymerase chain reaction (PCR),. PCR amplification reagents typically include, for example, an upstream primer, a downstream primer, a template nucleic acid, a thermostable polymerase enzyme, deoxyribonucleoside triphosphates (including dATP, dCTP, dGTP, TTP or dUTP), buffers, metal cations and salts. A nucleic acid sequence-based amplification (NASBA) reaction can include primers, reverse transcriptase, RNase H, and a DNA polymerase. A transcription-mediated amplification (TMA) reaction can include primers, reverse transcriptase, and an RNA polymerase. An strand displacement amplification (SDA) reaction can include a modified nucleotide and a restriction endonuclease. Certain amplification reactions can also include deoxyUridine-N-Glycosylase (UNG) as an ancillary amplification reagent.

### 9. Kits

In another aspect, the present invention provides kits comprising at least one probe comprising a releasable label moiety immobilized to a solid substrate, as described above, and reagents for an amplification reaction, as described above. The kits find use in the detection of a target nucleic acid in a sample. In certain embodiments, the thermostable DNA polymerase has reverse transcription activity. In certain embodiments, the kits comprise instructions for detecting a nucleic acid according to the methods of the invention. In other embodiments, the kits comprise one or more containers to hold the components of the kit.

In certain embodiments, the kits can contain a composition comprising a primer of the invention. The kits can also contain a composition comprising a probe of the invention. The kits can further contain a composition comprising a thermostable DNA polymerase. In some embodiments, the thermostable DNA polymerase is selected from the group of *Carboxydothermus hydrogenformans* DNA polymerase, *Thermosipho africanus* DNA polymerase, *Bacillus pallidus* DNA polymerase, *Thermus* species Z05 DNA polymerase, *Thermus aquaticus* DNA polymerase, *Thermus thermophilus* DNA polymerase, *Thermatoga maritima* DNA polymerase, *Thermatoga neapolitana* DNA polymerase and *Thermus* sps17 DNA polymerase. The compositions comprising a primer or probe of the invention or a thermostable DNA polymerase can further comprise additional reagents. For example, the compositions can comprise suitable preservatives to prevent degradation of the composition, suitable buffers to modulate the pH of the composition, suitable diluents to alter the viscosity of the compositions, and the like.

The kits can additionally comprise other reagents for carrying out a 5'-nuclease reactions, as described above. In addition, the kits can comprise reagents to facilitate the detection of a fragmented probe that indicates the presence of a target nucleic acid. Kits that can be used to detect a nucleic acid of defined sequence are described in U.S. Patent Nos. 6,514,736; 6,197,563; 6,040,166, and 5,641,864. One of skill in the art can easily use the primers and probes of the invention to modify the disclosures of these U.S. Patents to design additional kits that are also within the scope of the present invention.

### EXAMPLES

The following examples are offered to illustrate, but not to limit the claimed invention.

### Example 1:

### Probe and Primer Synthesis

Oligonucleotide primers were prepared on controlled-pore glass with an Applied Biosystems 394 DNA synthesizer using standard phosphoramidite chemistry (S. L. Beucage, Methods in Molecular Biology, Vol. 20: Protocols for Oligonucleotides and Analogs, pp. 33-61). Phosphoramidite analogs of deoxyadenosine, deoxycytidine, deoxyguanosine and thymidine were purchased from Applied Biosystems (Foster City, California) and diluted to 0.1 M in anhydrous acetonitrile before use. Coupling, oxidation and deprotection reagents were also purchased from Applied Biosystems and used according to the manufacturer's recommendations. t-Butylbenzyl-dA CPG was prepared as previously described *(see,* U.S. Patent No. 6,001,611). Oligonucleotide probes were prepared with dye-phosphoramidites from BioGenex (San Ramon, California), and 5-methyl-uridine phosphoramidite and 3'-amino-modifier C7 CPG (C₇NH₂) from Glen Research (Sterling, Virginia).

The following modified oligonucleotides were prepared and purified by ion-exchange HPLC:

### Immobilization in DNA-bind plate

The amino-modified oligonucleotide probe described above was diluted to 3 µM in 50 mM sodium phosphate and 1 mM EDTA (TE), pH 8.5, and added in 100 µL aliquots to the wells of a DNA-bind Thermowell microwell plate from (Corning Life Sciences, Ithaca, New York). The plate was covered and stored in the dark at 5 °C for 18 hours, and drained. The wells were rinsed three times with Dubecco's PBS, drained, filled with 200 µL each of a solution of 3% BSA (Sigma Chemicals, St. Louis, Missouri) in sodium phosphate/EDTA. The plate was warmed to 37 °C for 30 minutes, drained, and rinsed three times with 10 mM Tris**·**HCl and 0.1 mM EDTA, pH 8.0. The dry plate was wrapped in foil and stored at 5 °C.

### PCR conditions

PCR master mix, 950 µL, containing 50 mM Tricine, pH 8.3, 90 mM potassium acetate, 200 µM each of dATP, dCTP and dGTP, 400 µM dUTP, 4 mM magnesium acetate, 2.5 % glycerol, 45% poly rA buffer (Roche Molecular Systems, Pleasanton, California), 0.5 µM each of upstream and downstream primers and 2.5 µL of Z05 DNA polymerase (100 units) (Roche Molecular Systems, Alameda, CA) in enzyme storage buffer was combined with 50 µL of a solution of Molt 4 genomic DNA in TE, 20 ng/µL. Aliquots (100 µL) of the master mix were added to the wells of a DNA-bind plate containing immobilized probe.

The plate, containing master mix and immobilized probe and covered with an Applied Biosystems optical adhesive cover, was heated in an ABI Prism 7700 at 95 °C, 20 s and 58 °C, 40 s for 45 cycles, and cooled to 4 °C. Aliquots of the reaction mixtures were reserved for analysis by capillary electrophoresis (see below).

### Release of immobilized probe through base hydrolysis

The cycled PCR plate described above was rinsed with TE, and filled with 120 mL each of 20mM sodium hydroxide, 200 mM NaCl solution. The plate was sealed with an optical adhesive cover and warmed to 75 °C for 200 minutes. Aliquots of the reaction products were analyzed by capillary electrophoresis.

### Analysis

Aliquots (1µL) of the reaction mixtures described above were added to 10 µL of formamide (Applied Biosystems) containing 6-carboxy-fluorescein standard and analyzed on an ABI 310 Genetic Analyzer equipped with a 47 cm x 50 µm capillary.

### Results

Capillary electrophoresis (CE) of the amplification reaction mixture resulted in the display of probe fragments in the HEX channel only, indicating that the immobilized probe was partially digested from the 5'-end. The absence of significant signals in the FAM channel indicates that the 3'-end of the probe remains attached during the PCR process.

CE analysis of the probe hydrolysis reaction products resulted in the display of undigested probe, visible in the HEX and FAM channel, and probe fragments visible in the FAM channel only. The ratio of intact probe to digested probe is approximately 50% as indicated by the relative area of full-length vs. fragment peaks.

It is understood that the examples and embodiments described herein are for illustrative purposes only and that various modifications or changes in light thereof will be suggested to persons skilled in the art and are to be included within the spirit and purview of this application and scope of the appended claims.

## Claims

1. A method for detecting a target nucleic acid, the method comprising the steps of:
a) providing an oligonucleotide probe immobilized onto a solid substrate via its 3'-end, the probe comprising a label moiety;
b) amplifying a nucleic acid sample suspected of comprising the target nucleic acid in a reaction mixture containing an enzyme having 5'-3'-nuclease activity in the presence of the probe, wherein the probe specifically hybridizes to the target nucleic acid such that the enzyme having 5'-3'-nuclease activity releases the label moiety from the oligonucleotide probe and the presence or absence of the target nucleic acid in the nucleic acid sample is detected.

2. The method of claim 1, wherein the label moiety is a fluorophore moiety attached to the 5'-end of the probe, whereby release of the fluorophore moiety results in a decrease in the fluorescence on the immobilized probe, thereby detecting the presence of the target nucleic acid in the nucleic acid sample.

3. The method of claim 1, wherein the label moiety is a quencher moiety attached to the 5'-end of the probe and the immobilized probe further comprises a fluorophore moiety attached to the probe 3' from the quencher moiety, wherein the fluorescence of the fluorophore moiety is reduced when the quencher moiety is attached to the probe, and the fluorescence of the fluorophore moiety on the immobilized probe increases with increasing amplification of the target nucleic acid as the quencher moiety is released into solution.

4. The method of claim 1, wherein the label moiety is a quencher moiety attached to the 5'-end of the probe and the fluorescence of a fluorophore moiety immobilized adjacent to the probe increases with increasing amplification of the target nucleic acid.

5. The method of claim 1, wherein the enzyme having 5'-3'-nuclease activity is a polymerase having 5'-3'-nuclease activity.

6. The method of claim 1, wherein the solid substrate is selected from the group consisting of a multiwell plate, a microscope slide, a PCR tube and a disk.

7. The method of claim 1, wherein the amplification detection is concurrent with amplification or subsequent to amplification.

8. A method of simultaneously detecting multiple target nucleic acid sequences, the method comprising the steps of:
a) providing a plurality of oligonucleotide probes, each probe immobilized onto a solid substrate via its 3'-end, each probe comprising the same label moiety;
b) amplifying multiple nucleic acid samples suspected of comprising a target nucleic acid in a reaction mixture containing an enzyme having 5'-3'-nuclease activity in the presence of the probe, wherein each probe specifically hybridizes to one of the multiple target nucleic acids such that the enzyme having 5'-3'-nuclease activity releases the label moiety from the oligonucleotide probes and the presence or absence of the target nucleic acids in the nucleic acid samples is detected.

9. A reaction mixture comprising:
i) an oligonucleotide probe immobilized to a solid substrate via its 3'-end, the probe comprising a label moiety that is released by an enzyme having 5'-3'-nuclease activity; and
ii) reagents for amplification of a target nucleic acid, wherein the probe hybridizes to the target nucleic acid.

10. The reaction mixture of claim 9, wherein the label moiety is a fluorophore moiety attached to the 5'-end of the probe.

11. The reaction mixture of claim 9, wherein the label moiety comprises a quencher moiety attached to the 5'-end of the probe and the immobilized probe further comprises a fluorophore moiety attached to the probe 3' from the quencher moiety.

12. The reaction mixture of claim 9, wherein the label moiety is a quencher moiety attached to the 5'-end of the probe.

13. The reaction mixture of claim 9, wherein the immobilized probe hybridizes to a sequence on the target nucleic acid that is adjacent to a primer in the reagents for amplification.

14. The reaction mixture of claim 9, wherein the solid substrate is selected from the group consisting of a multiwell plate, a microscope slide, a PCR tube and a flat-surfaced disk.

15. The reaction mixture of claim 9, wherein the fluorophore moiety is selected from the group consisting of fluorescein-family dyes, polyhalofluorescein-family dyes, hexachlorofluorescein-family dyes, coumarin-family dyes, rhodamine-family dyes, cyanine-family dyes, oxazine-family dyes, thiazine-family dyes, squaraine-family dyes, chelated lanthanide-family dyes, and BODIPY^{®}-family dyes.

16. The reaction mixture of claim 9, wherein the quencher moiety is selected from the group consisting of fluorescein-family dyes, polyhalofluorescein-family dyes, hexachlorofluorescein-family dyes, coumarin-family dyes, rhodamine-family dyes, cyanine-family dyes, oxazine-family dyes, thiazine-family dyes, squaraine-family dyes, chelated lanthanide-family dyes, BODIPY^{®}-family dyes, azo-family dyes, triphenylmethane family dyes, low-fluorescent quencher moieties and non-fluorescent quencher moieties.

17. A kit comprising:
i) an oligonucleotide probe immobilized to a solid substrate via its 3'-end, the probe comprising a label moiety that is released by an enzyme having 5'-3'-nuclease activity; and
ii) reagents for amplification of a target nucleic acid, wherein the probe hybridizes to the target nucleic acid.

18. The kit of claim 17, wherein the solid substrate is selected from the group consisting of a multiwell plate, a microscope slide, a PCR tube and a flat-surfaced disk.

19. An apparatus for carrying out amplification of a target nucleic acid, the apparatus comprising an oligonucleotide probe immobilized to a solid substrate via its 3'-end, the probe comprising a label moiety that is released by an enzyme having 5'-3'-nuclease activity.
